# EUROPEAN PATENT APPLICATION

(11) **EP 2 883 964 A1**
(43) Date of publication of application: **17.06.2015**
(21) Application number: 13828279.3
(22) Date of filing: 08.08.2013
(51) Int. Cl.: C12Q 1/68, G01N 21/78, G01N 33/53, G01N 33/536

(54) **METHOD FOR LABELLING OF CLEAVAGE OF NUCLEIC ACID SEQUENCE**

(30) Priority: 09.08.2012 JP 2012176558
(71) Applicant: Japanese Foundation For Cancer Research, Tokyo 135-8550 (JP)
(72) Inventor: TAKEUCHI, Kengo, Tokyo 135-8550 (JP)
(74) Representative: EP&C
(86) International application number: PCT/JP2013/071576
(87) International publication number: WO 2014/025001

(57) **Abstract**

Probe sets which have been used in conventional FISH methods have a problem that the location of a labelled probe labelled with an orange fluorescent dye and the location of a labelled probe labelled with a green fluorescent dye is apart to some extent from each other and therefore both orange fluorescence and green fluorescence, rather than yellow fluorescence, are observed in some relative positions or angles between a fluorescence microscope and a target nucleic acid even when the target nucleic acid is not cleaved, which may lead to the false determination that the target nucleic acid is cleaved. [Solution] The following steps are involved: a step of bringing a hybridization solution into contact with a target nucleic acid (1), wherein the hybridization solution contains a probe set (5) that is composed of at least two labelled probes (5a, 5b) respectively labelled with different identifying factors and can hybridize with almost the full length of the sequence for the target nucleic acid (1); and a step of causing the identifying factors to develop the functions thereof to produce signals.

## Description

### FIELD OF TECHNOLOGY

The present invention relates to a method for labeling cleavages of nucleic acid sequences, whether or not a target nucleic acid sequence is cleaved.

### BACKGROUND ART

Fluorescence in situ hybridization (FISH method) and chromogenic in situ hybridization(CISH method) are known as methods for visually locating specific nucleotide sequences or genes.

FISH is a method whereby a cloned gene or DNA fragment, through a fluorescent dye, a labeled probe DNA is hybridized with a chromosomal DNA on a slide glass, and using a fluorescence microscope, the molecular hybridized part is detected directly on the chromosome as a fluorescent signal. Compared to conventional auto-radiographic methods, the FISH method is considered excellent because it does not utilize radioactive materials and therefore safe and convenient and results can be obtained in a short period of time. Likewise, CISH is a method whereby DNA and mRNA are detected on tissue specimens with the use of a pigment (chromogen) such as DAB, etc. Unlike the FISH method, the CISH method is characterized by the non-employment of fluorescent dyes, making observation possible under an optical microscope, thereby eliminating the need for a fluo-rescence microscope.

One application of the aforementioned FISH method pertains to the use of two or more kinds of probe DNAs labeled with a fluorescent dye emitting a color tone con-sisting of varying fluorescent colors (for example, orange, green, yellow, etc.), while mapping chromosomes at the same time.

A method for detecting cancer tissue by using the features of such type of a FISH technique has been proposed (for example, see Patent Documents 1 and 2). These detection methods involve a combination of a FISH technique with other detection methods, and have been shown to be effective in performing cancer diagnosis and treatment with a high degree of precision.

In addition, research and development of cancer diagnostic methods continues. In particular, examination of chromosomal abnormalities in cancer cells by way of reciprocal translocation, insertion, inversion, etc. is known. It is believed that such chromosomal abnormalities occur at the DNA level when segmentation and stringing of genes occurs and ordinarily genes that do not exist (fusion genes) are formed. Thus, specific fusion genes can be identified employing the FISH method, and through such detection, cancer diagnosis is made possible.

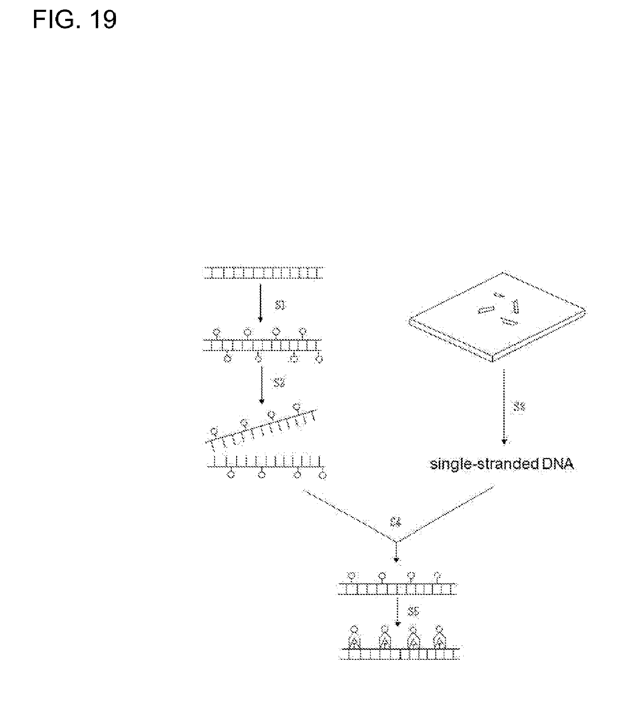

As a concrete example of the FISH method, a summary of a biotin labeled FISH method (indirect method) is shown in Figure 19. First, using biotin-dUTP (for example, Roche Applied Science, Inc.: Biotin-1-dUTP, etc.), a probe DNA is labeled (S1), to produce a biotin-labeled DNA. Then, the biotin-labeled DNA is thermally denatured (S2). At the same time, chromosomal specimens are also thermally denatured and single-stranded DNA are adjusted (S3).

Next, single-stranded DNA and biotin-labeled DNA are hybridized and complementary binding of each DNA is effected (S4). Then, after the resultant double-stranded biotin-labeled DNA and the chromosomal DNA have been washed, the biotin is treated with high affinity Avidin-FITC (for example, Roche Applied Science, Inc.: AP-labeled streptoavidin, etc.) solution (S5). After being washed with a pre-determined washing solution, the chromosomal DNA is fluorescently stained, and using a fluorescence microscope, the locus of a fluorescent signal on the chromosome is visualized. In this manner, the molecular hybridized part on the chromosome can be directly detected on the chromosome as a fluorescent signal.

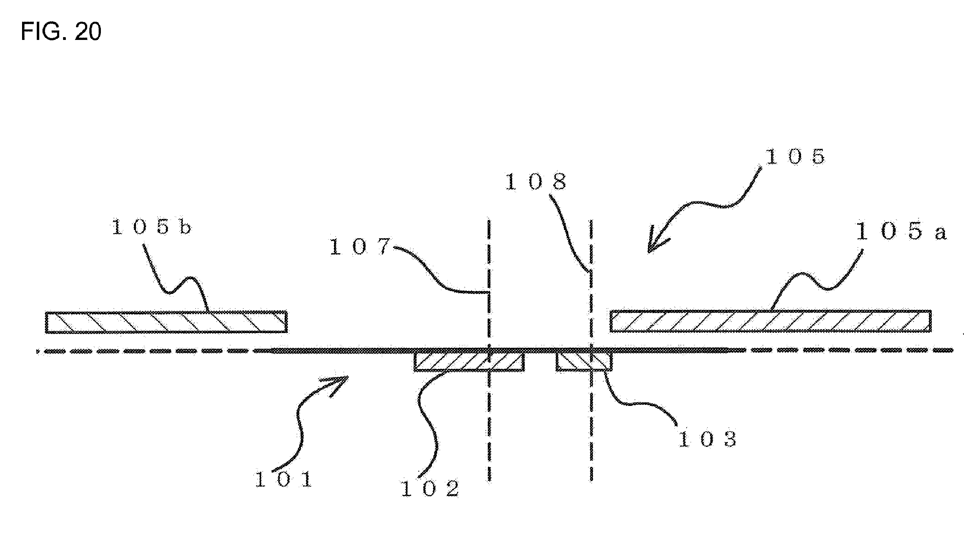

Hereafter, the probe sets employed in the FISH method involving two types of labels will be explained. As shown in Figure 20, usually, in the target DNA 101 used in the FISH method, the cleavage region 102 that includes the breakpoint of the target DNA101, for example, contains a pathophysiologically important key region 103. In the probe set 105 corresponding to the target DNA101, the two labeled probe sets 105a,105b hybridized with DNA are sandwiched in the cleavage region 102 of the target DNA101. The labeled probe 105a is labeled with an orange fluorescent dye and the labeled probe 105b is labeled with a green fluorescent dye. Further, the length of the target DNA101 is typically several times longer than the labeled probes 105a, 105b (by several hundred kb or more).

Using this probe set 105, the FISH method is employed under the environment of the existing target DNA101, and when a fluorescent signal is observed with the use of a fluorescence microscope, if the target DNA 101 is not cleaved, the DNA become close to each other as they are sandwiched in the cleavage region 102 of the target DNA 101. Then, the labeled probes 105a, 105b also become close to each other as they bind with the DNA sandwiched in the cleavage region 102 of the target DNA 101. As a result, when the uncleaved target DNA101 is observed under the fluorescence microscope, the yellow fluorescence produced by the orange fluorescence and the green fluorescence overlap is observed.

On the other hand, when the target DNA101 is cleaved and divided into two target DNA pieces by reciprocal translocation, etc., these target DNA pieces are frequently located in separate places such that the labeled probes 105a, 105b that have bound with DNA in the vicinity of the target DNA 101 likewise often become separated to a certain extent. As a result, when the cleaved target DNA is observed under a fluorescence microscope, the orange fluorescence hardly overlaps the green fluorescence so that the fluorescence of each can be separately observed, and either one of the target DNA pieces leaves the observation area and due to the absence and disappearance of one of the target DNA pieces, only the fluorescence of the labeled probe located in the vicinity of the remaining target DNA piece becomes visible.

Therefore, by using the conventional FISH method, when observing the target DNA101 that may be cleaved by reciprocal translocation, etc., basically, the orange fluorescence and green fluorescence, or either fluorescence together with the yellow fluorescence become observable.

The CISH method is similar to the FISH method as explained in the example above.

### PRIOR ART REFERENCES

### PATENT DOCUMENTS

Patent Document 1: Japanese Unexamined Patent Application Publication No. 2004-157053
Patent Document 2: Japanese Translation of PCT International Application Publica-tion No. JP-T-2002-542793

### SUMMARY OF THE INVENTION

### PROBLEMS TO BE SOLVED BY THE INVENTION

However, in the probe set 105 used in the conventional FISH method, as shown in Figure 20, each of the labeled probes 105a, 105b bind with the DNA sandwiched in the cleavage region 102 of the target DNA 101. Therefore, because the orange fluorescent dye as well as the green fluorescent dye become separated to a certain extent, even if the target DNA 101 is not cleaved, each of the orange fluorescence and the green fluorescence and not the yellow fluorescence is visible, depending on the position and angle of the fluorescence microscope relative to the target DNA 101.

Consequently, when analyzing the fluorescence obtained under a fluorescence microscope, since the orange fluorescence, the green fluorescence and the yellow fluorescence are observable, the uncleaved target DNA 101 is mistakenly believed to be cleaved, and the possibility of falsely perceiving the distance between the labeled probe 105a and the labeled probe 105b increases. This problem equally applies with respect to the CISH method.

Also, in the probe set 105 used in the conventional FISH method, the issue of whether the target DNA101 is cleaved will be determined by the respective position of and distance between the orange fluorescence and the green fluorescence. Accordingly, in the probe set 105, as shown in Figure 20, the problem of whether the target DNA101 is cleaved at the breakpoint 107 forming part of the cleavage region 102 or at the breakpoint 108 not included in the cleavage region 102 cannot be resolved.

This is a major problem that arises when the FISH method is used for example, in cancer diagnosis. That is to say, when this type of probe set 105 is used in conducting the FISH method for diagnosing cancer whether it is the fluorescence of the cleaved target DNA pathophysiologically due to a significant cleavage (for example, a cleavage corresponding to the breakpoint 107 forming part of the cleavage region 102), or the fluorescence of the target DNA pathophysiologically due to a significant thin cleavage (for example, a cleavage corresponding to the breakpoint 108 not included in the cleavage region 102) being observed cannot be determined. Accordingly, although the outcome of cancer diagnosis is actually negative, the same is erroneously judged as positive.

Further, while the orange fluorescence and the green fluorescence have been observed with low probability even if the target DNA is not cleaved, instruction manuals for probe sets which are commercially available also indicate it is preferable that the handling user establish the criteria for determining the extent in terms of percentage whether the target DNA is cleaved or not.

Considering the above-described circumstances attending the employment of either the FISH method or the CISH method, the present invention aims to provide a method for labeling cleavages of target nucleic acids to prevent an uncleaved target DNA from being mistakenly perceived as a cleaved DNA regardless of the relative position and angle of the fluorescence microscope vis-a-vis the target DNA when such fluorescence or the color of a chromogen is observed.

### MEANS FOR SOLVING THE PROBLEM

Owing to continuous diligent research in relation to this problem, the inventor of this invention has discovered a thorough method for labeling cleavages of target nucleic acids, described as follows.

To solve the above-mentioned problem, the first embodiment of the invention provides a method for labeling a cleavage of a nucleic acid sequence, characterized by a method for determining whether a target nucleic acid sequence having a cleavage region containing at least one breakpoint is cleaved or not, and comprising two or more labeled probes labeled with different identifying factors, a step of bringing a hybridization solution into contact with a target nucleic acid wherein the hybridization solution contains a probe set capable of hybridizing almost the entire sequence of the target nucleic acid, and a step for making identifying factors function to effect the emission of a signal.

According to the first embodiment, because two or more probe sets labeled by different identifying factors hybridize almost the entire sequence of a target nucleic acid, a signal from at least two different overlapping identifying factors in an uncleaved target nucleic acid (for example, under the FISH technique, when two fluorescent dyes such as orange and green are used, a yellow fluorescence) becomes visible.

On the other hand, in the case of the cleaved target nucleic acid split into nucleic acid fragments, for example, the resultant blending of at least two different identifying factors (for instance, under a FISH technique, when two fluorescent dyes such as orange and green are used, a yellow fluorescence) and the signal of either identifying factor (for instance, under a FISH technique, when two fluorescent dyes such as orange and green are used, either the orange fluorescence or the green fluorescence) of the two fluorescences becomes visible, or, either one of the target nucleic acid fragments leaves the observation area and due to the absence and disappearance of one of the target nucleic acid fragments, the signal of the identifying factor of the remaining target nucleic acid fragment (for instance, under a FISH technique, when two fluorescent dyes such as orange and green are used, the orange fluorescence, or the green fluorescence or the yellow fluorescence) is observable.

In the case where the target nucleic acid is not cleaved, when observing yellow fluorescence through a filter or the like (including image processing technology), orange fluorescence and green fluorescence is visible in the vicinity of the yellow fluores-cence.

Therefore, if the first embodiment of the present invention is employed, the signal of the blended identifying factors in the uncleaved target nucleic acid is visible, while in the case of the cleaved target nucleic acid, in contrast to the conventional FISH technique, because the signal of the blended identifying factors and either signal of two signals of the identifying factors is observable, or one of the signals of the identifying factors or the signal of the blended identifying factors becomes visible, misreading the uncleaved target nucleic acid for being cleaved can be prevented.

Moreover, because the probe set hybridizes substantially the entire sequence of the target nucleic acid, by comparing the quantity of the signal (in terms of color, intensity, etc.) of the identifying factors of the uncleaved target nucleic acid with the quantity of the signal of the identifying factors of the cleaved target nucleic acid, estimation of the cleaving position of the target nucleic acid is made possible.

The second embodiment of the present invention provides for a method of labeling the cleavage of a nucleic acid sequence according to the first embodiment characterized in that at least one labeled probe hybridizes the cleavage region of the said nucleic acid sequence.

According to the second embodiment, aside from the effect obtainable in the aforesaid first embodiment, a clear determination of whether the nucleic acid sequence is cleaved at the cleavage region or at some other location becomes possible.

The third embodiment of the present invention provides for a method of labeling the cleavage of a nucleic acid sequence according to the second embodiment characterized in that the nucleic acid sequence further comprises at least one key region and different labeled probes comprising one labeled probe that hybridizes the cleavage region of the nucleic acid sequence, and relative to the cleavage region, a labeled probe that hybridizes a portion of the nucleic acid sequence sandwiched in the key region.

According to the third embodiment, it is possible to determine whether the nucleic acid sequence is cleaved not at the critical region of the breakpoint or whether the nucleic acid sequence is cleaved at some other location.

The fourth embodiment of the present invention provides for a method of labeling a nucleic acid sequence according to any one of the first to the third embodiments characterized in that a probe set comprises a fusion region labeled with different identifying factors in a blended condition.

According to the fourth embodiment, if the target nucleic acid is cleaved and split into target nucleic acid fragments, and hybridized with a probe set, the signal of either one of the labeling factors in at least one of the target nucleic acid fragments, together with the signal of the blended (overlapping) identifying factors from the fusion region of the probe set passing through a filter, also becomes observable. On the other hand, although it is possible that a nucleic acid other than the target nucleic acid may be hybridized by the labeled probe, only the signal of the labeling factor bound to the said labeled probe from the said nucleic acid can be visualized.

Therefore, by using the fourth embodiment of the present invention, even when the labeled probe hybridizes a nucleic acid other than the target nucleic acid, when the signal of the blended identifying factors from the fusion region is confirmed, the said signal would indicate whether the target nucleic acid is cleaved, and because it is possible to determine that such nucleic acid is other than the target nucleic acid, the uncleaved target nucleic acid being mistaken for a cleaved nucleic acid is more reliably averted.

The fifth embodiment of the present invention provides for a method of labeling a nucleic acid sequence according to according to any one of the first to the fourth embodiment characterized in that the fusion region hybridizes with the key region in its entirety or a portion thereof.

According to the fifth embodiment, the question of whether a nucleic acid sequence is cleaved at the breakpoint included in the cleavage region or is cleaved at some other location can be adjudged with greater certainty.

The sixth embodiment of the present invention provides for a method of labeling a nucleic acid sequence according to any one of the first to the fifth embodiments characterized in that single or multiple labeled probes labeled with the same identifying factor hybridize almost the entire sequence of the target nucleic acid.

According to the sixth embodiment, because the target nucleic acid is cleaved at a portion corresponding to a labeled portion at an identical identifying factor, by comparing the signal amount of the identifying factor of the uncleaved target nucleic acid with the signal amount of the identifying factor of the cleaved target nucleic acid, the cleaving position of the target nucleic acid can be estimated with greater accuracy.

Also, since the target nucleic acid is not cleaved at a region corresponding to the boundary of each identifying factor, the signal of each blended identifying factor in the case of the uncleaved target nucleic acid is visualized, while the signal of any identifying factor and the signal of each blended identifying factor in the case of the cleaved target nucleic acid is observed, or, because any one of those signals is observable, the uncleaved target nucleic acid being mistaken for a cleaved nucleic acid is more reliably prevented.

The seventh embodiment of the present invention provides for a method of labeling a nucleic acid sequence according to any one of the first to the sixth embodiments characterized in that different identifying factors are different color dyes, and a pro-cess for making the identifying factors function as to emit a signal is the process for making the said dyes radiate light to develop colors, and a process for detecting a signal is the process for detecting a developed color.

According to the sixth embodiment, it is possible to carry out the method of labelling a cleavage of a nucleic acid sequence in a manner similar to the conventional CISH method.

The eighth embodiment of the present invention provides for a method of labeling a nucleic acid sequence according to any one of the first to the sixth embodiments characterized by different identifying factors comprising fluorescent dyes that emit dissimilar fluorescent colors, a process for causing fluorescent dyes to produce fluorescence by making identification factors function to emit a signal, whereby the process for detecting a signal is the process for detecting fluorescence.

According to the eighth embodiment, it is possible to carry out the method of labelling a cleavage of a nucleic acid sequence in a manner similar to the conventional FISH method.

The ninth embodiment of the present invention provides for a method of labeling a nucleic acid sequence according to either the seventh or the eighth embodiments characterized in that it comprises a colored dye developed from two different hues in the modified Munsell transformation color system or a fluorescent dye emitting two different fluorescent hues in the modified Munsell transformation color system, whereby a region of the breakpoint of the target nucleic acid is labeled by a large angle formed by a straight line connecting a color from among these different hues and the epicenter of a modified Munsell hue circle, and a straight line connecting a color mixture resulting from the fusion of two different hues to the epicenter of a modified Munsell hue circle, whereby the large angle comprises a color dye or a fluorescent dye and such region of the breakpoint corresponds to a nucleic acid sequence.

According to the ninth embodiment, a straight line connecting a hue to the epicenter of a modified Munsell hue circle, and a straight line connecting a color mixture resulting from the fusion of two different hues to the epicenter of a modified Munsell hue circle, constitute an angle (a hue angle) comprising a color dye or a fluorescent dye, whereby the distinction between the color dye or fluorescent dye of a large hue angle and the said color mixture is more easily recognized than the distinction between the color dye or fluorescent dye of a small hue angle and the said color mixture. Thus, the signal of each blended identifying factor in the case of the uncleaved target nucleic acid is visualized, while the signal of any identifying factor and the signal of each blended identifying factor in the case of the cleaved target nucleic acid can be observed, and for this reason, mistaking the uncleaved target nucleic acid for being a cleaved nucleic acid is certainly avoided further.

As used in the present invention, the term "nucleic acid" refers to deoxyribonucleic acid (DNA), ribonucleic acid (RNA), peptide nucleic acid (PNA) and those having functions similar to these nucleic acids. The sequence and length of the nucleic acids of the present invention are not restricted to those of the said nucleic acids, as the term includes even those nucleic acids that currently exist in the natural world, as well as those that are produced by mutation (for example, a fusion gene, etc.), or artificially created. Solid phase synthesis can be cited as an example of a method for synthesizing nucleic acids artificially.

Likewise, to a person carrying out the present invention, the term "cleavage region" refers to a portion of an assumed nucleic acid sequence which includes a breakpoint, but refers to the entire target nucleic acid when the breakpoint is unknown.

Further, to a person carrying out the present invention, the term "key region" refers to the demand determining portion of a nucleic acid sequence, for instance, when implementing the present invention as a method for diagnosing cancer, pathophysiologically, it is a region of a prominent nucleic acid (for example, kinase domain, etc.). Still, in the present invention, there must be at least one key region in a nucleic acid sequence although two or more key regions may be present in the nucleic acid sequence. Further still, if a plurality of key regions exist in a nucleic acid sequence, it would be appropriate for a person carrying out the present invention to regard the key region as an important part of the present invention.

### BRIEF DESCRIPTION OF THE DRAWINGS

Figure "1" is a conceptual view of a probe set according to the first embodiment.
Figure "2" is a conceptual view of a cleaved target nucleic acid.
Figure "3" is a conceptual view of a hybridized probe set in a cleaved target nucleic acid according to the first embodiment.
Figure "4" is a conceptual view of a probe set according to the first embodiment.
Figure "5" is another conceptual view of a probe set according to the first embodiment.
Figure "6" is another conceptual view of a hybridized probe set in a cleaved target nucleic acid according to the first embodiment.
Figure "7" is a conceptual view of a probe set according to the first embodiment.
Figure "8" is a conceptual view of a labeled probe according to the second embodiment.
Figure "9" is a conceptual view of a probe set according to the second embodiment.
Figure "10" is a conceptual view of a hybridized probe set in a cleaved target nucleic acid according to the second embodiment.
Figure "11" is a conceptual view of a probe set according to the third embodiment.
Figure "12" is another conceptual view of a probe set according to the third embodi-ment.
Figure "13" is a conceptual view of a probe set according to the fourth embodiment.
Figure "14" is another conceptual view of a probe set according to the fourth embodi-ment.
Figure "15" is another conceptual view of a probe set according to the fourth embodi-ment.
Figure "16" is a conceptual view of a probe set according to an alternative embodiment.
Figure "17" is a fluorescent image view of a working example.
Figure "18" is a fluorescent image view of a comparative working example.
Figure "19" is a schematic illustration of a FISH technique.
Figure "20" is a conceptual view of a conventional probe set.

### MODES FOR CARRYING OUT THE INVENTION

The present invention provides for a method for labeling a cleavage of a nucleic acid sequence, comprising two or more labeled probes labeled with different identifying factors, and a hybridizing solution including a probe set that can hybridize substantially the entire sequence of the said target nucleic acid, and two processes consisting of a step for of bringing the target nucleic acid in contact with a hybridization solution including a probe set capable of hybridizing almost the entire sequence of the target nucleic acid (Step 1), and a step for making the said identifying factors function to emit a signal (Step 2). Each step is described hereafter. Note that these steps, except for the configuration of the probe set, can be implemented in a manner similar to conventional FISH and CISH techniques and the like.

First, Step 1 will be explained. The hybridization solution used in Step 1 comprises two or more labelled probes labeled with different identifying factors, and provided that a probe set that is capable of hybridizing almost the entire sequence of the target nucleic acid is included, Step 1 is not particularly restricted.

Here, there is no particular restriction as to what would constitute an identifying factor as it may comprise, for example, a fluorescent dye employed in a FISH technique or a pigment used in a CISH technique, or depending on some other kind of stimuli, light (including ultraviolet rays, infrared rays) that radiates or is transmitted (including an absorbed portion of a wavelength).

Likewise, there is no particular restriction as what would constitute a labeled probe, provided it can hybridize the target nucleic acid and is labeled by a labeling factor. For instance, deoxyribonucleic acid (DNA), ribonucleic acid (RNA), peptide nucleic acid, and such other nucleic acids having similar functions labeled by labeling factors, and such sequences and the length thereof are not particularly restricted, as well as other nucleic acids actually existing in the natural world or even artificially created.

It should be noted that DNA or the like that is labeled with a labeling factor may comprise even DNA that is directly bound to an identifying factor (DNA bound through a direct process), or DNA that is indirectly bound to an identifying factor (DNA bound through an indirect process) through protein, antibodies and the like (including DNA stacked on several layers).

Then, a probe set that is deemed capable of hybridizing substantially the entire sequence of a target nucleic acid, is a probe set that can hybridize the entire sequence of the target nucleic acid not only completely, but also deal with a portion of the entire sequence of the target nucleic acid that cannot be hybridized. Below is a description of such probe set.

Accordingly, a hybridization solution including such type of a probe set, for instance, even those commercially available reagents for hybridization containing other compo-nents utilized in FISH or CISH techniques and such other techniques are acceptable.

The probe set used in the present invention will be described in relation to the FISH technique involving the use of two kinds of fluorescent dyes as an example. Similarly, the probe set of the present invention may be conceived in respect of the CISH tech-nique.

### EMBODIMENT 1

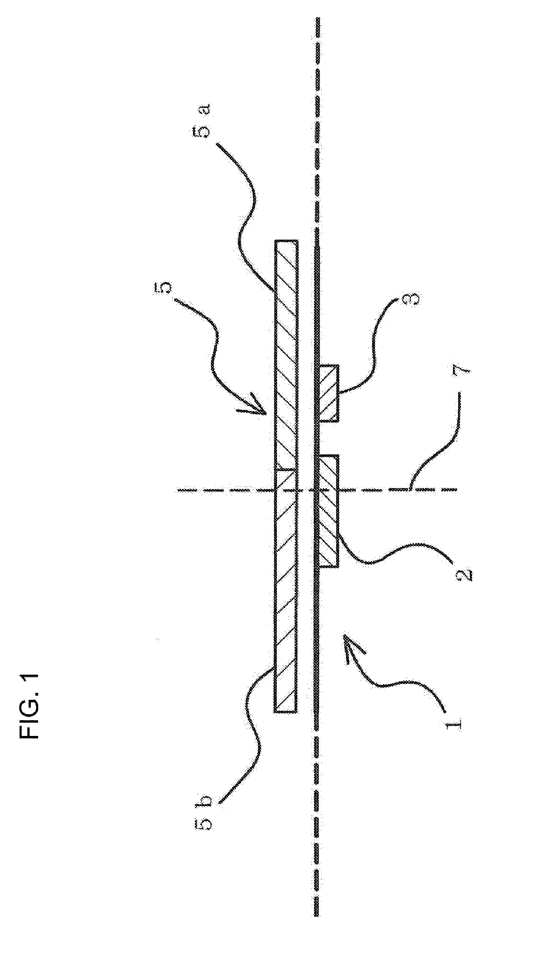

Figure 1 is a schematic view of the probe set 5 hybridizing a target nucleic acid 1 containing a cleavage region 2 with at least one breakpoint 7 and a key region 3. As shown in Figure 1, the probe set 5 used in the present invention is composed of the labeled probes 5a, 5b, but in contrast with the probe set used under the conventional FISH technique, the probe set 5 can hybridize the entire sequence of the target nucleic acid 1. Then, the labeled probe 5a is labeled with a fluorescent dye with orange fluorescence while the labeled probe 5b is labeled with a fluorescent dye with green fluorescence.

Thus, after the target nucleic acid 1 and probe set 5 are hybridized, the second step described below is performed, such that when the fluorescent dyes are rendered fluorescent, each of the labeled probes 5a, 5b labeled with fluorescent dyes become closer to each other, such that the orange fluorescence of the labeled probe 5a and the green fluorescence of the labeled probe 5b overlap, and yellow fluorescence is observed. However, in the vicinity of the yellow fluorescence, orange fluorescence and green fluorescence can likewise be observed.

Here, the probe set 5 is designed to be capable of hybridizing with the target nucleic acid 1. For example, as shown in Figure 1, when the probe set 5 hybridizes with the target nucleic acid 1, the two labeled probes 5a, 5b are designed in such a way that no portion thereof will overlap with the target nucleic acid 1.

It must be noted that although complementarity between the labeled probes 5a, 5b and the sequence of the target nucleic acid 1 is preferably 100%, this is not a restriction as long as they retain the ability to specifically bind to the target nucleic acid 1. Even complementarity of less than 100% in some embodiments of the target nucleic acid, for instance, 90%, is suitable. It is also preferable for the labeled probes 5a, 5b to contain identical glycine (G) / cytosine (C) ratios so as to have similar levels of thermal stability. This combination of labeled probes 5a, 5b may also be structurally similar to those commercially available, or those newly prepared, or those artificially produced by the solid phase synthesis method.

Next is an explanation of Step 2. Step 2 is not particularly restricted provided that the identifying factors are made to function in order to emit a signal. For instance, when the identifying factor is a fluorescent dye, using a laser device and the like, capable of making each fluorescent dye emit fluorescent wavelengths of light, causing the fluorescence dye of the labeled probes 51, 5b to become fluorescent is sufficient. Moreover, when the identifying factor is a fluorescent dye, it is sufficient for the dye of the probe sets to develop color by using a device and the like capable of emitting visible light.

Then, the signal obtained from an identifying factor may be observed through a fluorescent microscope and the like or an observation instrument. There is no restriction with respect to the observation instrument provided that it is capable of detecting the signal obtainable in accordance with Step 2. For example, when the identifying factor is a fluorescent dye, in commercially available flourescence microscope devices used in conventional FISH techniques, such as optical devices, digital imaging hardware, computer hardware and computer software, the fluorescence of the identifying factor i s detectable. Likewise, when the identifying factor is a dye, in commercially available optical microscope devices employed in conventional CISH techniques, such as optical devices, digital imaging hardware, computer hardware and computer software, the color of the identifying factor is detectable.

Here, the signal observed in the case of an uncleaved target nucleic acid and a cleaved target nucleic acid will be described in relation to the FISH technique referred to and explained in Step 1.

As mentioned above, when the target nucleic acid 1 is not cleaved, yellow fluorescence is observable when the orange fluorescence blends with the green fluorescence.

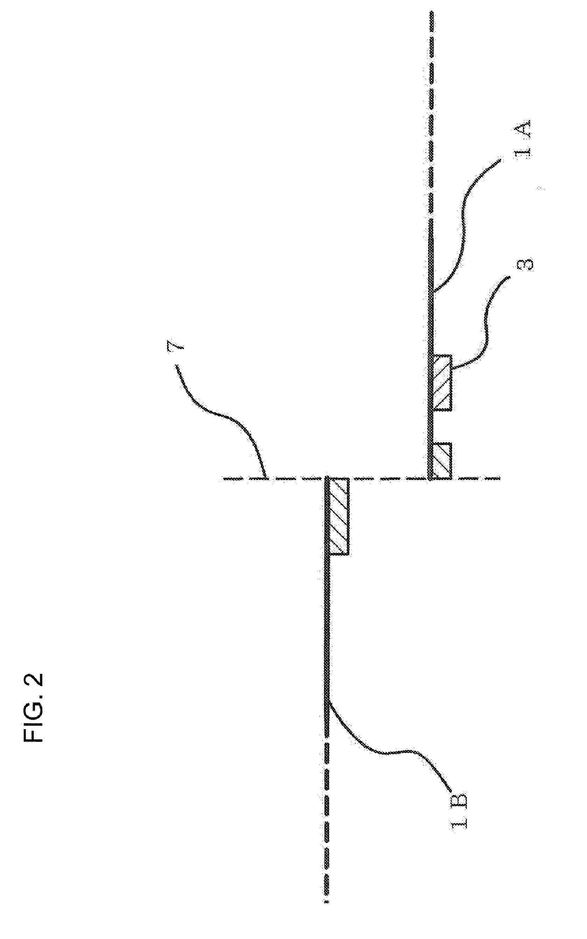

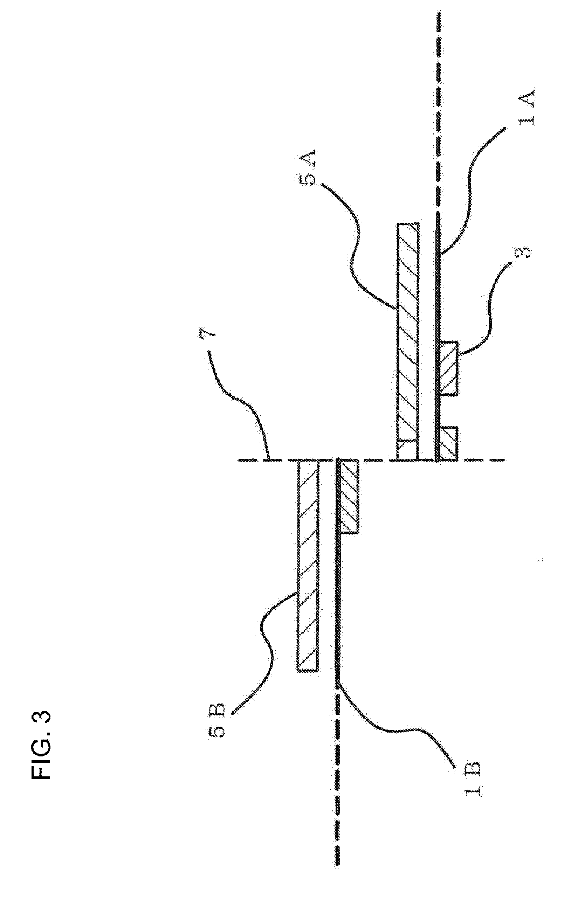

On the other hand, for instance, due to the influence of mutual translocation, as illustrated in Figure 1, when the target nucleic acid 1 is cleaved at the breakpoint 7 included in the cleavage region 2, as shown in Figure 2, the target nucleic acid 1 is divided into target nucleic acid fragments 1A, 1 B. Then, conducting Step 1 as shown in Figure 3, the probe set 5 hybridizes each of the said target nucleic acid fragments 1A, 1 B and each of the apparently cleaved probe set pieces 5A, 5B at the breakpoint 7. The probe set piece 5A is labeled with orange fluorescent dye and green fluorescent dye, while the probe set piece 5B is labeled only with green fluorescent dye. And, these target nucleic acid fragments 1A, 1 B are often located apart from each other.

As a result, when the target nucleic acid 1 is cleaved and divided into target nucleic acid fragments 1A, 1 B, the orange fluorescence of the probe set piece 5A (and a small amount of green fluorescence overlapping a portion thereof to produce yellow fluores-cence) and the green flourescence of the probe set piece 5B are observed, while either one of the target nucleic acid fragments 1A, 1 B leaves the observation area and due to the absence and disappearance of one of the target nucleic acid fragments 1A, 1 B, only one fluorescence becomes visible.

Thus, unlike the conventional FISH method, only the yellow fluorescence of the probe set 5 is observed when the target nucleic acid 1 is not cleaved, and the orange fluorescence of the probe set piece 5A (and a small amount of green fluorescence overlapping a portion thereof to produce a yellow fluorescence) and the green fluorescence of the probe set piece 5B are observed, or, only either one of the two fluorescences becomes visible in the case where the target nucleic acid 1 is cleaved, such that an erroneous determination that the uncleaved target nucleic acid 1 is a cleaved nucleic acid can be prevented.

Again, since the probe set 5 can hybridize the entire sequence of the target nucleic acid 1, by comparing the color and intensity of the fluorescence of the uncleaved target nucleic acid 1, with the color and intensity of the cleaved target nucleic acid fragments 1A, 1 B, estimating the breakpoint 7 of the target nucleic acid 1 is possible.

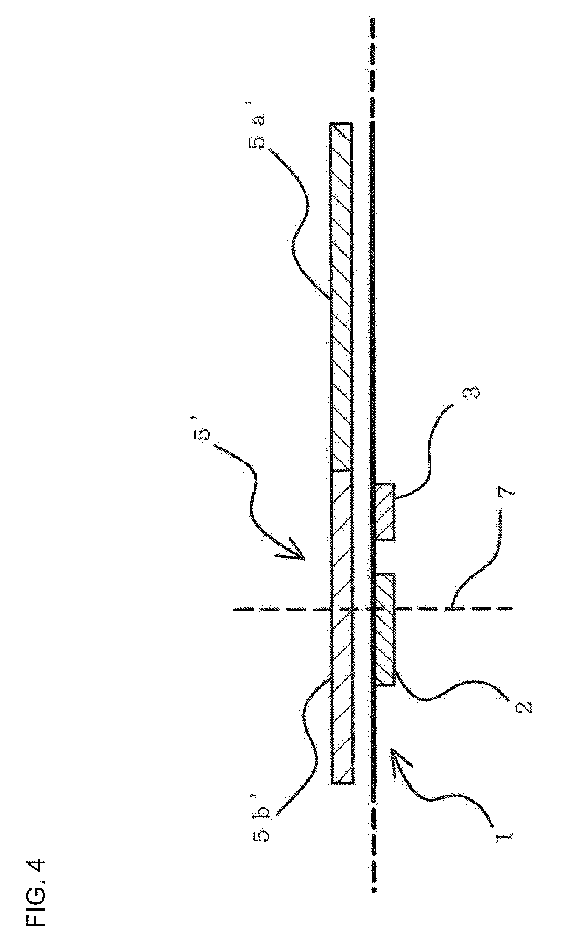

Notably, like the probe set 5 shown in Figure 1, the probe set not only comprises different labeled probes consisting of a labeled probe 5b that hybridizes the cleavage region 2 of the target nucleic acid 1, and relative to the cleavage region 2, a labelled probe 5a that hybridizes a portion of the nucleic acid sequence 1 sandwiched in a key region 3, but, like the probe set 5" shown in Figure 4, the effect mentioned above likewise occurs when the probe set also comprises identical labeled probes consisting of a labeled probe 5b' that hybridizes the cleavage region 2 of the target nucleic acid 1, and relative to the cleavage region 2, a labeled probe 5b' that hybridizes a portion of the target nucleic acid 1 sandwiched in the key region 3 (where the labeled probe 5b' is relatively longer than the labeled probe 5b).

Furthermore, in this example, as described above, a hybridizing probe set 5' consists of a labeled probe 5a' and a labeled probe 5b' that hybridizes the cleaved target nucleic acid 1 at the breakpoint 7 in the cleavage region of the target nucleic acid. Accordingly, thereafter, when the fluorescent dye is made to fluoresce, orange fluorescence (and a small amount of the green fluorescence overlapping a portion thereof to produce yellow fluorescence) and green fluorescence is observed (see Fig. 4).

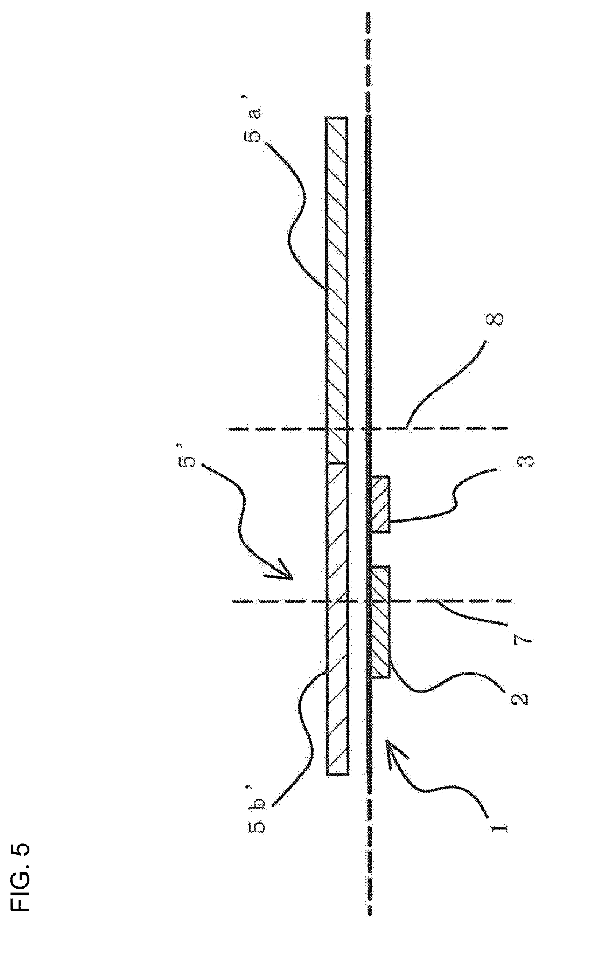

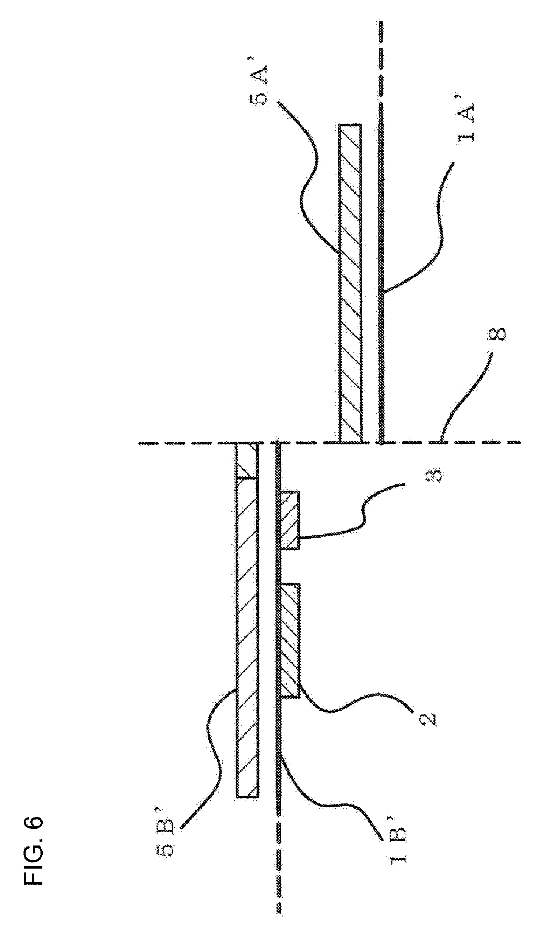

On the other hand, if for some reason, the target nucleic acid that is not usually cleaved is cleaved at the breakpoint 8 not included in the cleavage region 2, it splits into target nucleic acid fragments 1A', 1 B' as shown in Figure 5, and the probe set 5' hybridizes each of the apparently cleaved probe set pieces 5A', 5B' at the breakpoint 8 as shown in Figure 6. Then, under this condition, when the fluorescence dyes are made to fluoresce, the orange fluorescence of the probe set piece 5A' and the green fluorescence of the probe set piece 5B' (a portion of the orange fluorescence overlaps the green fluorescence to produce a yellow fluorescence) are observed.

Therefore, by observing the fluorescence in the present embodiment, the issue of whether the target nucleic acid 1 is cleaved at the breakpoint 7 contained in the cleav-age region 2 or the breakpoint 8 not contained in the cleavage region 2 can be resolved.

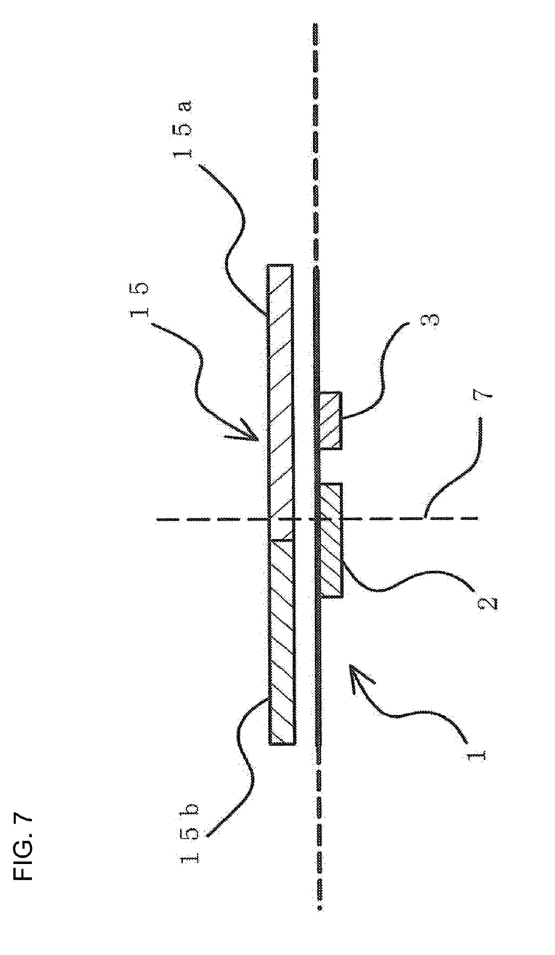

Further, as shown in Figure 7, the probe set 15 may also be configured in that the labeled probe 15a hybridizes a portion of the target nucleic acid sandwiched in the cleavage region 2 relative to the key region 3, and the labeled probe 15b hybridizes the cleavage region 2 in the key region 3, such that the arrangement of each labeled probe is reversed in relation to the labeled probes of probe set 5 illustrated in Figure 1.

If the probe set 15 is configured in the above-described manner, when the target nucleic acid 1 is cleaved in the breakpoint 7, the orange fluorescence of the labeled probe that hybridized a portion of the target nucleic acid that does not contain the key region 3 (and a portion of the orange fluorescence that overlaps the green fluorescence producing yellow fluorescence), and the green fluorescence of the labeled probed that hybridized a portion of the target nucleic acid 1 that contains the key region 3, are obsesved.

Therefore, when the probe set 15 is configured in this manner, by observing the green monochromatic fluorescence, an interpretation that a significant cleavage (for example, when this invention is carried out as a method for cancer diagnosis, pathophysiologically a significant cleaveage) has materialized can be made.

### EMBODIMENT 2

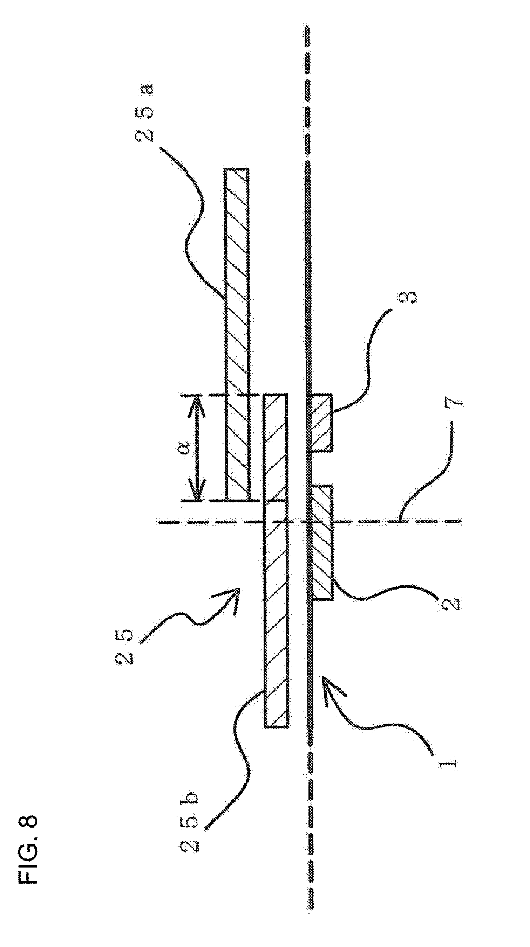

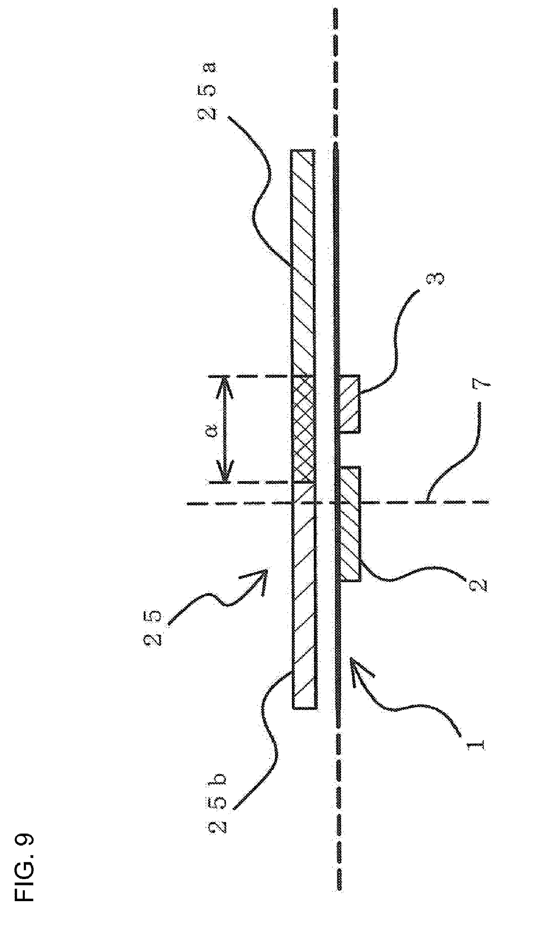

In the first embodiment, although the probe set 5 is designed in a manner that no portion of the two labeled probes 5a, 5b overlaps the target nucleic acid 1 as shown in Figure 1, it may also be designed in such a way that the two labeled probes 25a, 25b overlap to create the fusion region α when the target nucleic acid 1 hybridizes the probe set 25, as shown in Figure 8. Typically, the fluorescent dyes will be labeled in a blended condition if the probe set 25 is structured in this manner with a fusion region α.

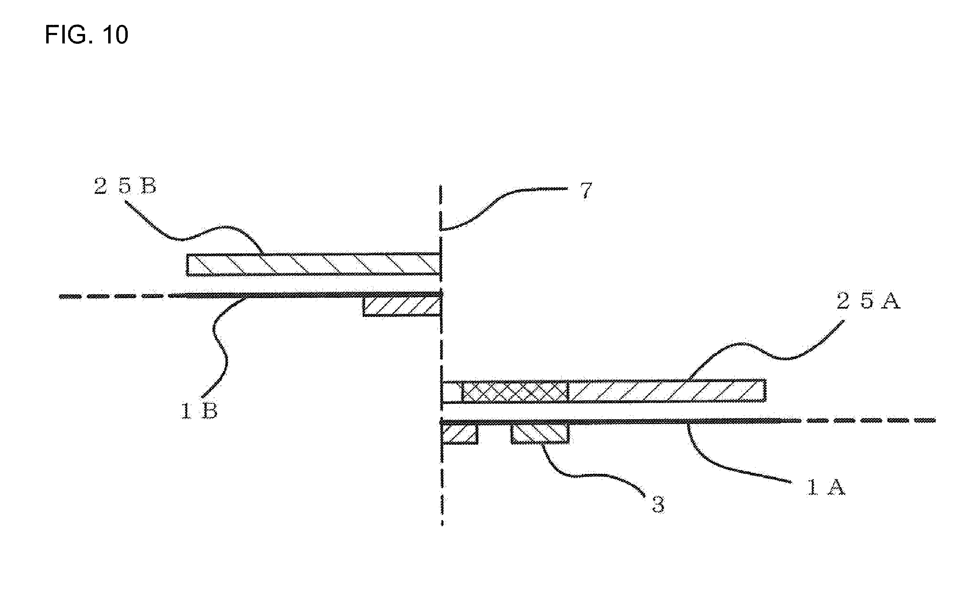

Then, using this probe set 25, the above mentioned Step 1 is conducted in respect of the cleaved target nucleic 1, such that the probe set 25 hybridizes each of the target nucleic acid fragments 1A, 1 B and the apparently cleaved probe set pieces 25A, 25B respectively at the breakpoint 7, as shown in Figure 10.

Thus, when the target nucleic acid is cleaved and splits into target nucleic acid fragments 1A, 1 B, the orange fluorescence, yellow fluorescence and green fluorescence of the probe set piece 25A overlap entirely, and the orange fluorescence or a part thereof which overlaps the green fluorescence producing yellow fluorescence as well as the green fluorescence of the probe set piece 25B are observed, and be-cause one of the target nucleic acid fragments 1A, 1 B leaves the observation area and due to the absence and disappearance of one of the target nucleic acid fragments 1A, 1 B, only one fluorescence becomes visible.

Notably, in the second embodiment, since the fusion region α constitutes a probe set 25 formed at a location corresponding to the key region 3, by confirming the existence of yellow fluorescence corresponding to the fusion region α next to the orange fluorescence, the question of whether the target nucleic acid 1 is cleaved at the breakpoint 7 or at some other location can be more reliably determined, making such configuration of the probe set 25 preferred. However, the probe set according to the second embodiment is not limited to this configuration.

In addition, by observing the probe set piece 25A through the use of a filer or image processing technology in a more detailed manner, the yellow fluorescence of the fusion region α can be observed together with the orange fluorescence and the green fluorescence. Thus, whether the target nucleic acid is cleaved at the breakpoint 7 included in cleavage region 2 or at some other location can be adjudged with more certainty when the presence of these fluorescences is confirmed.

### EMBODIMENT 3

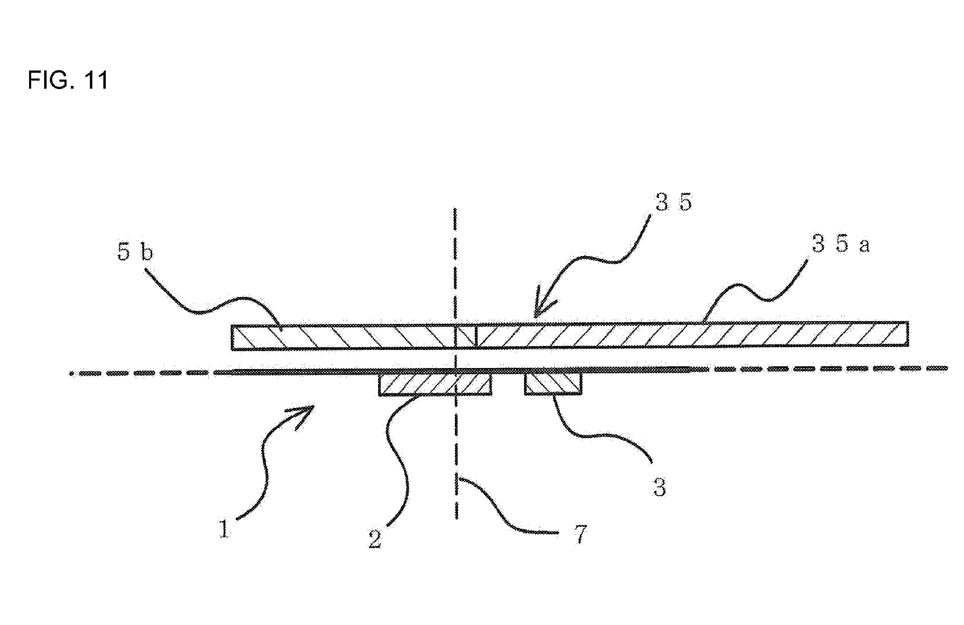

Although the probe set 5 of the Embodiment 1 comprises two labelled probes 5a, 5b which match the length of the entire sequence of the target nucleic 1, the length of the probe set of the current invention is not restricted thereto. The labeled probe 35a and labeled probe 35b comprising a labeled probe 35 of the target nucleic acid may be longer than the labeled probe 5a of Embodiment 1 as shown in Figure 11. The effect obtainable in the case of the probe set 5 of Embodiment 1 is attainable with the labeled probe 35 configured in this manner.

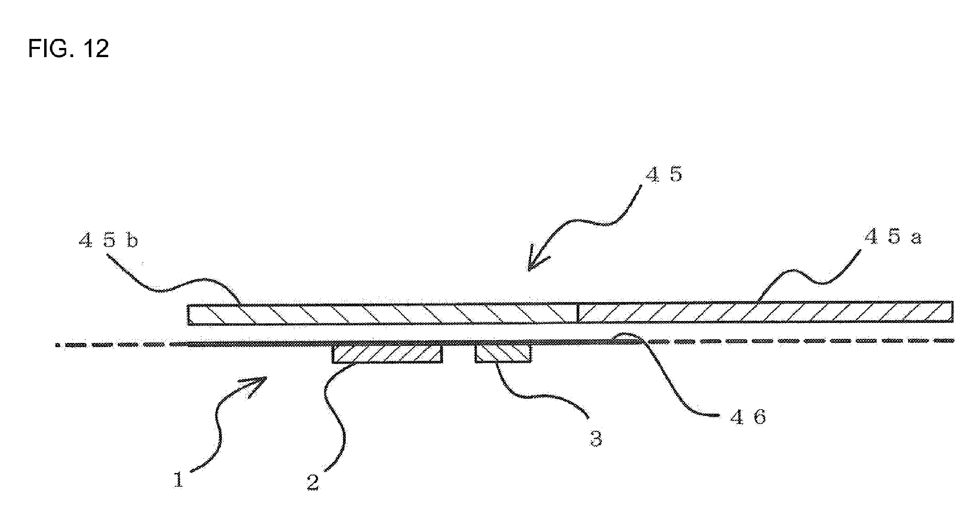

Moreover, as shown in Figure 12, configuring the labeled probe 45 to comprise labeled probe 45a that is longer than the labeled probe 5a of Embodiment 1, and labeled probe 45b containing the hybridized remaining portion 46 of the target nucleic acid 1, and which is capable of hybridizing almost the entire sequence of the target nucleic acid 1, is suitable. Notably, configuring the labeled probe 45b to comprise a nucleic acid sequence even longer than the target nucleic acid 1 is appropriate.

Even if the probe set 45 is configured in this manner, the effect obtained in the case of the probe set 5 of Embodiment 1 is similarly attainable and since the length of the labeled probe 45b is comparable with the length of the target nucleic acid 1, by measuring the intensity of the fluorescence of the labeled green dye and comparing the intensity of the fluorescence of the labeled green dye of the uncleaved target nucleic acid 1 with the intensity of the fluorescence of the green fluorescent dye of the target nucleic acid 1 when it is cleaved, the cleaving position of the target nucleic acid 1 can be estimated with greater accuracy.

### EMBODIMENT 4

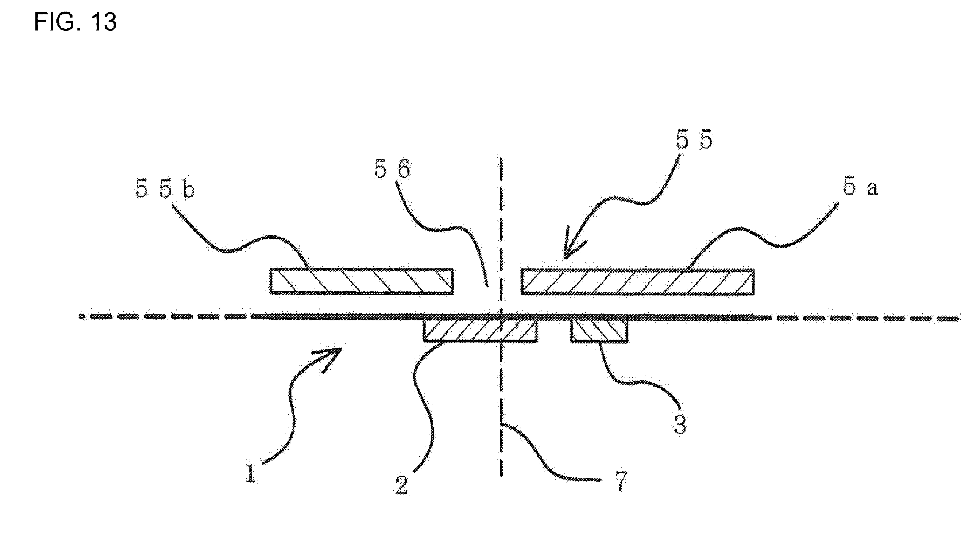

Unlike the probe set 5 of Embodiment 1 that can hybridize the entirety of the nucleic acid sequence, as shown in Figure 13, a probe set 55 can be configured to comprise a labeled probe 55b with a sequence shorter than that of the labeled probe 5a and labeled probe 5b but is capable of hybridizing the region corresponding to the cleavage region 2 that cannot be hybridized by the region 56.

In the probe set 55 of the said configuration, when the target nucleic acid 1 is hybridized by the probe set 55, space between the labeled probe 5a and the labeled probe 55b is created, thereby enabling the region 56 that cannot hybridize the target nucleic acid 1 to hybridize substantially the entire sequence of the target nucleic acid 1, and this being the case, the effect obtained in the case of the probe set 5 of Embodiment 1 is likewise attainable.

There is no restriction to the length of the region 56 which is not capable of hybridization as long as the effects of the present invention can be obtained, but is preferably 100kb or less, and increasingly desirable if the length is 50kb or less, 40kb or less, 30kb or less, or 20kb or less, and most preferably, 10kb or less. The present invention becomes more remarkably effective as the region 56 incapable of hybridization becomes shorter.

Also, when an investigation is to be made whether the known target nucleic acid 1 is cleaved by the breakpoint 7, as shown in Figure 13, the labeled probe set 55 may also be configured to consist of labeled probe 5a and labeled probe 55b in order that the region 56 incapable of hybridizing the target nucleic acid 1 is able to do so in the vicinity of breakpoint 7.

In accordance with the said configuration of the probe set 55, although yellow fluorescence formed by the blending of orange fluorescence and green fluorescence is observable in the case of the uncleaved target nucleic acid 1, each of the orange fluorescence and green fluorescence become separately visible when it is cleaved, and therefore, misreading the uncleaved target nucleic acid as a cleaved nucleic acid can be prevented with greater certainty.

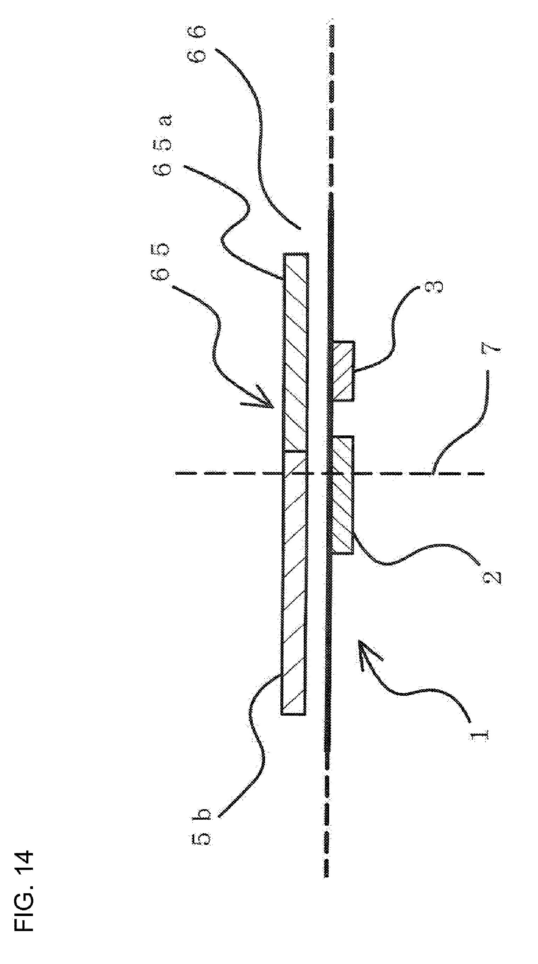

In this example, although the probe set 55 is of such a configuration, in order to allow space between the labeled probe 5a and the labeled probe 55b, as shown in Figure 14, the probe set 65 may be configured to comprise the labeled probe 5b and the labeled probe 65a with sequence even shorter than that of the labeled probe 5a, in order that the region 66 incapable of hybridization becomes capable of hybridizing the end of the target nucleic acid 1 when the probe set 55 hybridizes the target nucleic acid 1. The effect obtained in the case of probe set 5 of Embodiment 1 can be similarly achieved by a probe set 65 of this configuration.

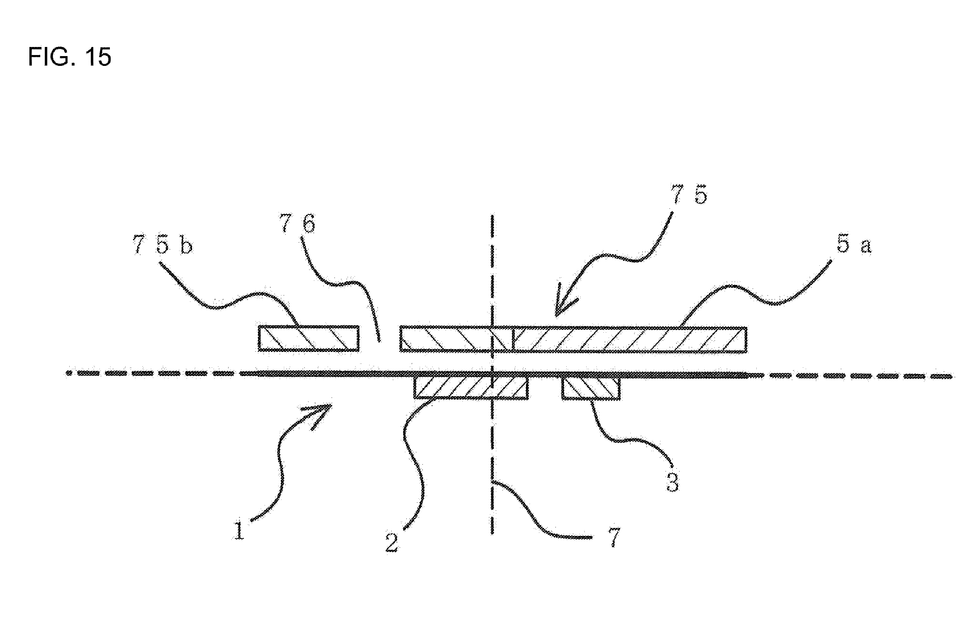

Furthermore, as shown in Figure 15, a suitable probe set 75 may consist of the labeled probe 5a and a labeled probe 75b lacking a region 76 of the labeled probe 5b of Embodiment 1. If there is a region in the labeled probe 5b that can hybridize a nucleic acid other than the target nucleic acid 1, by configuring the probe set 75 to comprise the labelled probe 75b without such region and the labeled probe 5a, it is possible to suppress the fluorescence of a nucleic acid sequence other than that of the nucleic acid 1, and this being the case, misreading the uncleaved target nucleic acid 1 as being cleaved is more reliably prevented.

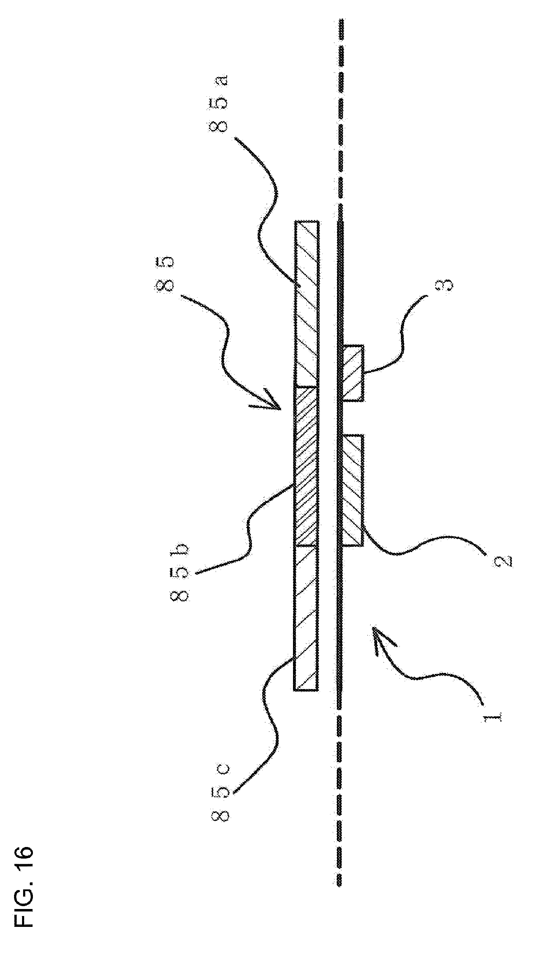

Although the probe set described above comprises a probe set consisting of two labeled probes, the probe set of the present invention is not limited to this configuration, as it may consist of several labeled probes, for example, as shown in Figure 16, a probe set 85 comprising three labeled probes 85a, 85b, 85c. The effect obtained in the probe set 5 of Embodiment 1 is similarly obtainable in a probe set 85 of this configuration.

Notably, in the case of each of the labeled probes 85a, 85b, 85c, the use of fluorescent dyes coupled with each of the labeled probes 85a, 85b, 85c emitting varying fluorescences is appropriate, for instance, the fluorescent dye of the labeled probe 85b may be similar to the fluorescent dye of either that of the labeled probe 85a or the fluorescent dye of the labeled probe 85c (the entire probe set 85 comprises fluorescent dyes of two hues). When the probe set 85 comprising the labeled probes 85a, 85b, 85c, is employed where varying fluorescences are emitted by the fluorescent dye in each of the said labeled probes 85a, 85b, 85c, the positional of the target nucleic acid 1 hybridizing each of the said labeled probes becomes more obvious, and consequently, compared to the probe set 5 of Embodiment 1, greater accuracy in estimating the cleaving position of the target nucleic acid 1 can be achieved.

While other embodiments of the probe set have been discussed above, the configuration of the probe sets of the present invention are not limited thereto, as a probe set may be further configured to include a combination of the same.

If the identifying factor comprises a color development of two different hues in the modified Munsell transformation color system (for example, orange and green) or a fluorescent dye which emits two different fluorescent hues in the modified Munsell transformation color system (for example, orange and green), it is preferable to design a probe set whereby a region of the breakpoint of the target nucleic acid is labeled by an angle (a hue angle) formed by a straight line connecting one color from among these different hues and the epicenter of the modified Munsell transformation color system and a straight line connecting a color mixture resulting from the fusion of two different hues to the epicenter of a modified Munsell transformation color system, where a large hue angle comprises a color dye (for example, green) or a fluorescent dye (for example, green) and such region of the breakpoint corresponds to a nucleic acid sequence.

If a large hue angle comprises a color dye (for example, green) or a fluorescent dye (for example, green) and a small hue angle comprises a color dye (for example, orange) or a fluorescent dye (for example, orange), the distinction between the color dye or fluorescent dye of a large hue angle and the said color mixture is more easily recognized than the distinction between the color dye or fluorescent dye of a small hue angle and the said color mixture. Consequently, the signal of each blended identifying factor in the case of the uncleaved target nucleic acid is visualized, while the signal of any identifying factor and the signal of each blended identifying factor in the case of the cleaved target nucleic acid can be observed, and for this reason, a false interpretation of the uncleaved target nucleic acid as being a cleaved nucleic acid can be avoided with greater certainty.

### EXAMPLE

To detect the re-configuration of an ALK gene, an RP11-984I21 labeled with FITC, and an RP11-701P18 labeled with RP11-62B19 and TexRed were employed as labeled probes, and after hybridization, a procedure similar to a conventional FISH technique was performed, and a fluorescent image was created. It should be noted that RP11-984I21, RP11-62B19 and RP11-701P18 are identification numbers at the human male RP-11 BAC library.

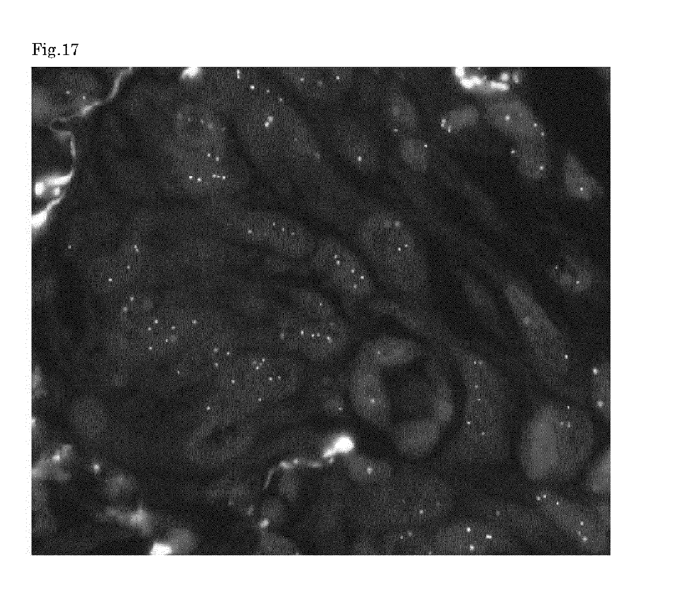

A fluorescence image in relation to the Example (a negative example) is illustrated in Figure 17. As can be gleaned from this figure, in all fluorescences, orange fluores-cence and green fluorescence overlap to produce yellow fluorescence which is visualized. (In the vicinity of yellow fluorescence, orange and green fluorescence are visible.) Here, "negative" is perceived without the occurrence of reciprocal translocation, and a target nucleic acid is in an uncleaved state. "Positive" on the other hand refers to a situation where translocation occurs and a target nucleic acid is in a cleaved state. Notably, when a labeled probe was labeled with FITC and TexRed in reverse position, the same effect was obtained.

### COMPARATIVE EXAMPLE

On the other hand, in order to detect the re-configuration of ALK gene, an LSI ALK Dual Color was used as an identifying probe, and after hybridization, the conventional FISH technique used in the procedure employed in the Example was performed, and a fluorescent image was created.

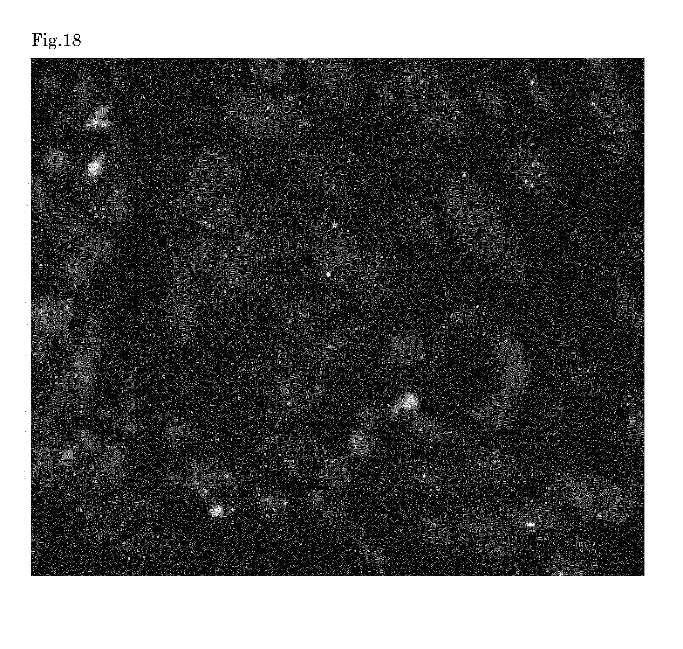

A fluorescence image in relation to the Comparative Example (a negative example) is illustrated in Figure 18. As can be gleaned from this Figure, orange fluorescence and green fluorescence are separately visible from a portion of a fluorescence. Therefore, the target nucleic acid is visualized as being cleaved and as such, the possibility of judging the result as "positive" exists. This is due to the fact that in the conventional probe set, orange fluorescence of a fluorescent dye and green fluorescence of a fluorescent dye are located apart from each other to a certain extent and for this reason, even if the target nucleic acid is not cleaved, depending on the relative position of the fluorescence microscope and the target nucleic acid, it is likely that orange fluorescence and green fluorescence can be observed.

As explained above, by using the present invention, a misreading of the uncleaved target nucleic acid as being a cleaved nucleic acid can be averted.

### INDUSTRIAL APPLICABILITY

The invention can be applied in conventional FISH techniques which are employed as diagnostic methods (including cancer diagnostic methods), as well as DNA or RNA identification methods, or methods for identifying the particular mode of DNA or RNA, and also as a method for estimating the cleaving position of nucleic acids.

### DESCRIPTION OF SYMBOLS

- 1,101: Target nucleic acid
- 1A, 1 B, 1A', 1B': Target nucleic acid fragment
- 2, 102: Cleavage region
- 3, 103: Key region
- 7, 8, 107, 108: Breakpoint
- 5, 15, 25, 35, 45, 55, 65, 75, 85, 105: Probe set
- 5A, 5B, 5A', 5B', 25A, 25B: Probe set pieces
- 5a, 5b, 5a', 5b', 15a, 15b, 25a, 25b, 35a,:
- 45a, 45b, 55b, 65a, 75b, 85a, 85b, 85c, 105a, 105b: Labeled probes
- α: Fusion region

## Claims

1. A method of labeling a cleavage of a nucleic acid sequence by distinguishing whether a target nucleic acid sequence having a cleavage region containing at least one breakpoint is cleaved or not, **characterized by**
two or more labeled probes labeled through different identifying factors,
a step of bringing a hybridization solution into contact with the said target nucleic acid,
wherein the hybridization solution contains a probe set capable of hybridizing almost the entire sequence of the target nucleic acid, and
a step for making identifying factors function to effect the emission of a signal.

2. A method of labeling the cleavage of a nucleic acid sequence according to Claim 1 **characterized in that** at least one labeled probe of the said labelled probes hybridizes the cleavage region of the said nucleic acid sequence.

3. A method of labeling the cleavage of a nucleic acid sequence according to Claim 2 **characterized in that**
the said nucleic acid sequence comprises at least one key region and
two different labeled probes, one of which hybridizes the cleavage region of the said nucleic acid sequence, and
relative to the said cleavage region, a labeled probe that hybridizes a portion of the said nucleic acid sequence sandwiched in the said key region.

4. A method of labeling a nucleic acid sequence according to any one of Claims 1 to 3 **characterized in that** the said probe set comprises a fusion region labeled with different identifying factors in a blended condition.

5. A method of labeling a nucleic acid sequence according to Claim 4 **characterized in that** the said fusion region hybridizes with the said key region in its entirety or a portion thereof.

6. A method of labeling a nucleic acid sequence according to any one of Claims 1 to 5 **characterized in that** single or multiple labeled probes labeled with the same identifying factor hybridize almost the entire sequence of the said target nucleic acid.

7. A method of labeling a nucleic acid sequence according to any one of Claims 1 to 6 **characterized in that** said different identifying factors are dyes of different colors,
wherein a process for making the said identifying factors function as to emit a signal is the process for making the said dyes radiate light to develop colors, and
a process for detecting the said signal is the process for detecting a developed color.

8. A method of labeling a nucleic acid sequence according to any one of Claims 1 to 6 **characterized in that** said different identifying factors are fluorescent dyes which emit dissimilar fluorescent colors,
wherein a process for causing the said identifying factors to function as to emit signals is the process for causing the said fluorescent dyes to fluoresce, and
a process for detecting said signals is the process for detecting said fluorescences.

9. A method of labeling the said nucleic acid sequence according to either Claim 7 or Claim 8, **characterized in that**
the said colored dyes are developed from two different hues in the modified Munsell transformation color system or the fluorescent dyes emit two different fluorescent hues in the modified Munsell transformation color system,
wherein a region of the breakpoint of the target nucleic acid is labeled by a large angle comprising a colored dye or a fluorescent dye formed by a straight line connecting a color from among the said different hues and the epicenter of a modified Munsell hue circle, and a straight line connecting a color mixture resulting from the fusion of two different hues to the epicenter of a modified Munsell hue circle, and such region of the breakpoint corresponds to a nucleic acid sequence.
